# EUROPEAN PATENT APPLICATION

(11) **EP 1 867 735 A1**
(43) Date of publication of application: **19.12.2007**
(21) Application number: 07109792.7
(22) Date of filing: 07.06.2007
(51) Int. Cl.: C12Q 1/68, B01L 7/00

(54) **A method and apparatus for judging the presence or absence of metastasis of malignant tumor**

(30) Priority: 13.06.2006 JP 2006163833; 28.12.2006 JP 2006355544
(71) Applicant: SYSMEX CORPORATION, Kobe-shi, Hyogo 651-0073 (JP)
(72) Inventor: Nakabayashi, Kazuki, Kobe-shi Hyogo 651-0073 (JP); Otomo, Yasuhiro, Kobe-shi Hyogo 651-0073 (JP); Daito, Motonari, Kobe-shi Hyogo 651-0073 (JP); Takata, Hideki, Kobe-shi Hyogo 651-0073 (JP); Hiyama, Kayo, Kobe-shi Hyogo 651-0073 (JP)
(74) Representative: De Clercq, Ann G. Y.

(57) **Abstract**

A method and apparatus for judging the presence or absence of metastasis of malignant tumor is herein described. The method comprising: measuring an absolute amount of a tumor marker; and judging the presence or absence of metastasis of malignant tumor by comparing the absolute amount with a predetermined threshold value. The apparatus comprising: a measuring assembly for obtaining information related to an absolute amount of a tumor marker; and a computer for obtaining absolute amount of the tumor marker based on the information related to the absolute amount of the tumor marker, and judging the presence or absence of metastasis by comparing the absolute amount of the tumor marker with a predetermined threshold value.

## Description

### TECHNICAL FIELD

The present invention relates to a method and apparatus for detecting metastasis of malignant tumor.

### BACKGROUND

In order to test metastasis of malignant tumor with respect to a particular tissue, currently, molecular test of malignant tumor using the LAMP (loop mediated isothermal amplification) method or the PCR (polymerase chain reaction) method is vigorously studied. A molecular test can be performed by detecting a tumor marker (e.g. mRNA of a protein specifically expressed in an oncocyte) contained in a tissue or a cell. For example, it is known that, as the tumor marker for determining metastasis of a breast cancer to a lymph node (hereinafter, also referred to as marker simply), mRNA of cytokeratin 19 (CK19) or carcinoembryonic antigen (CEA) is useful. Expression level (an amount of expression) of a tumor marker in a normal lymph node and an expression level of a tumor marker in a breast cancer cell metastasized to a lymph node are significantly different.

A specimen used in determining metastasis of malignant tumor can be collected by biopsy, and the number of contained cells is different depending on a specimen. Upon determination of metastasis by a molecular test, each specimen containing the different number of cells is used for detecting a tumor marker.

The conventional method for determining metastasis is performed as follows:
an amount of a tumor marker in a specimen is measured;
a measured amount of a tumor marker is normalized;
a normalized amount of a tumor marker is compared with a predetermined threshold value; and
metastasis is determined using this comparison result.

Normalization can be performed, for example, by dividing an amount of the tumor marker in a specimen by an expression level of a housekeeping gene which is thought to be expressed at an approximately constant amount in any cell regardless of a kind of a cell (e.g. Inokuchi et al., British Journal of cancer (2003) 89,1750-1756). Thereby, a value indicating how many tumor markers are expressed per one molecule of the expression product of the housekeeping gene can be calculated. Therefore, regardless of the number of cells contained in a specimen, a normalized value of the tumor marker can be compared with a particular threshold value or a normalized value of the tumor marker of other specimen.

### SUMMARY

The conventional determination method is thought to be on assumption that cells contained in a specimen are homogeneous (all cells in a specimen to be analyzed are substantially the same kind of cells). For example, when almost of cells in a sample are oncocytes, this can be deemed as homogeneous system.

However, a specimen actually collected from a living body contains not only oncocytes but also normal cells in many cases. For this reason, the present inventors found out a problem that, when expression level of tumor marker is normalized, malignant tumor metastasis can not be correctly determined in some cases. Then, a method and an apparatus which solve such the problem, and enable erroneous determination to be suppressed minimally were developed, resulting in completion of the present invention.

The scope of the present invention is defined solely by the appended claims, and is not affected to any degree by the statements within this summary.

A first aspect of the present invention relates to a method for judging the presence or absence of metastasis of malignant tumor, comprising steps of:
measuring an absolute amount of a tumor marker in the tissue or in a cell of the tissue obtained from a living subject; and
judging the presence or absence of metastasis of malignant tumor to the tissue by comparing the absolute amount with a predetermined threshold value.

A second aspect of the present invention relates to an apparatus for judging the presence or absence of metastasis of malignant tumor, comprising:
a measuring assembly for obtaining information related to an absolute amount of a tumor marker in the tissue or in a cell of the tissue obtained from a living subject; and
a computer for obtaining absolute amount of the tumor marker based on the information related to the tissue by comparing the absolute amount of the tumor marker with a predetermined threshold value.

### BRIEF DESCRIPTION OF THE DRAWINGS

Fig. 1 is a perspective showing an entire construction of the apparatus of the present embodiment.
Fig. 2 is a perspective showing an entire construction of a nucleic acid amplification device as a measurement means shown in Fig. 1.
Fig. 3 is a schematic plane view of the nucleic acid amplification device of Fig. 2.
Fig. 4 is a flowchart showing a processing by CPU 102d of the personal computer 102.
Fig. 5 shows the mRNA copy number of each sample for measurement (result of Example 1).
Fig. 6 shows the normalized result obtained by dividing a copy number of CK19mRNA obtained in Example 1 by a copy number of mRNA of β-actin obtained in Comparative Example 1.
Fig. 7 shows the mRNA copy number of each sample for measurement (result of Example 2).
Fig. 8 shows the mRNA copy number of each sample for measurement (result of Example 3).
Fig. 9 shows the mRNA copy number of each sample for measurement (result of Example 4).
Fig. 10 shows the mRNA copy number of each sample for measurement (result of Example 5).
Fig. 11 shows the mRNA copy number of each sample for measurement (result of Example 6).

### DESCRIPTION OF THE PREFERRED EMBODIMENTS

In the method for detecting metastasis of malignant tumor of the present embodiment, a sample containing a tissue or a cell collected from a living body is used as a specimen. Examples include lymph node tissue, blood, urine, wash obtained by peritoneoclysis, and a concentrate thereof.

According to the present embodiment, metastasis of a malignant tumor to the tissue can be detected. And, when a sample containing a cell is used as a specimen, metastasis of the malignant tumor to a tissue to which the cell belongs can be determined. For example, when wash obtained by peritoneoclysis is used as a specimen, since this wash contains cells dropped from stomach by washing, metastasis of the malignant tumor to stomach can be detected.

Herein, "detecting metastasis" includes qualitative determination of the presence or the absence of metastasis, semi-quantitative determination of an extent of metastasis (e.g. determination as negative, positive or strongly positive), and quantitative determination of an extent of malignant tumor metastasis (e.g. determination of a size of metastasis, the number of metastasized malignant tumor cells). According to the present embodiment, these detection results are provided as information about metastasis.

As used herein, the "tumor marker" refers to a molecule whose expression level in an oncocyte is significantly more than an expression level in a normal cell. In the present embodiment, the tumor marker includes a nucleic acid, a protein and the like. Preferably, the tumor marker is a nucleic acid such as mRNA and DNA, more preferably mRNA.

Examples of a kind of the tumor marker include mRNA of cytokeratin (CK) such as CK18, CK19 and CK20, mRNA of Calcinoembryonic antigen (CEA), mRNA of MUC1 mucin, and mRNA of mammaglobin (MMG). The tumor marker is preferably mRNA of CK, more preferably CK19mRNA.

In the present embodiment, an absolute amount of a tumor marker is measured. As used herein, the "absolute amount of a tumor marker" means a value based only on an amount of the tumor marker, and is a value in which other factor in measurement, for example, a value based on an amount of a tissue or a cell is not taken into consideration. That is, this value is not normalized to the aforementioned housekeeping gene. Examples of the absolute amount of a tumor marker include a concentration, a molecule number (copy number) and the like of the tumor marker. In addition, as used herein, the "absolute amount of a tumor marker" includes "information related to an absolute amount of a tumor marker."

"Information related to the absolute amount of a tumor marker" is a value which varies based only on the absolute amount of a tumor marker, and can be various values as exemplified below, depending on a method of measuring the tumor marker. For example, the information can be a fluorescent intensity, turbidity, or an absorbance of a reaction solution, or a time or a PCR cycle number until these values reach a predetermined value.

Hereinafter, the absolute amount of a tumor marker is referred to as "measured value" of the tumor marker in some cases. In addition, obtaining of the absolute amount of a tumor marker is referred to as "measure a tumor marker" in some cases.

In the method of the present embodiment, it is preferable to measure the tumor marker using a sample for measurement prepared by treating the tissue or the cell using a treatment solution. It is preferable that the treatment solution contains dimethyl sulfoxide (DMSO). When the tumor marker is measured using a nucleic acid amplification method, inhibition of the nucleic acid amplification reaction by an inhibitor contained in a sample can be reduced by the action of DMSO. By performing such the treatment on a tissue or a cell, the tumor marker contained in the tissue or the cell can be transferred into a solution.

The concentration of DMSO in the treatment solution is preferably 5 to 30% by volume, more preferably 10 to 25% by volume of the treatment solution.

The treatment solution may contain a buffer and a surfactant.

A pH of the treatment solution is preferably 2.5 to 5.0. It is preferable that a buffer such as a glycine-hydrochloric acid buffer is contained in order to maintain a pH at 2.5 to 5.0.

The surfactant is not particularly limited as far as it is a surfactant which is usually used in the art. Preferably, the surfactant is a nonionic surfactant, more preferably a polyoxyethylene-based nonionic surfactant. Particularly, a polyoxyethylene-based nonionic surfactant represented by the following general formula is suitable.

R1-R2- (CH₂CH₂O) ₙ-H

(wherein R1 is an alkyl group, an alkenyl group or an alkynyl group of a carbon number of 10 to 22, or an isooctyl group; R2 is-O- or -(C₆H₄)-O-; n is an integer of 8 to 120)

Examples of the surfactant include polyoxyethylene lauryl ether, polyoxyethylene cetyl ether, polyoxyethylene oleyl ether, polyoxyethylene myristyl ether, polyoxyethylene stearyl ether, polyoxyethylene nonyl phenyl ether, and polyoxyethylene isooctyl phenyl ether. Particularly, Brij 35 (polyoxyethylene (35) lauryl ether) is preferable. A concentration of the surfactant is not particularly limited as far as it is usually used in the art. The concentration is preferably 0.1 to 6% by volume, more preferably 1 to 5% by volume of the treatment solution.

The surfactant has an action of damaging a cell membrane to solubilize a tissue or a cell. By using the treatment solution containing such the surfactant, the tumor marker present in a cell membrane can be effectively transferred into a solution.

By using the aforementioned treatment solution, a sample for measurement can be prepared simply and in a short time without extraction and purification of a nucleic acid which are generally performed using a commercially available purification kit.

A ratio of mixing the treatment solution and a tissue or a cell is not particularly limited. When the tissue is used, the treatment solution may be added at around 0.0001 to 0.005mL per 1mg of the tissue, followed by mixing.

It is preferable that, after the treatment solution and the tissue or the cell are mixed, the tissue or the cell is crushed. Examples of the crushing method include homogenization with a homogenizer and crushing with an ultrasound crushing machine. As the homogenizer, a homogenizer which is usually used in the art can be used. Examples include Waring blender, Potter-Elvehjem-type homogenizer, Polytron-type homogenizer, Dounce-type homogenizer, and the like. Alternatively, homogenization may be performed manually using a pestle.

A solution obtained by crushing by the aforementioned method can be crudely purified using a normal purification method such as centrifugation, filtration, and column chromatography. This may be further purified by the known nucleic acid extracting method depending on a kind of the tumor marker to be detected.

In the present embodiment, a sample for measurement can be prepared by subjecting the tissue or the cell to the aforementioned treatment, purification or the like.

In the present embodiment, the tumor marker can be measured according to the method which is usually used in the art.

When the tumor marker is a protein, the tumor marker can be measured by the conventional method such as Western blotting method, Radioimmunoassay, Enzymatic immunoassay, and the method described in Japanese Patent Application Laid-Open (JP-A) No.2003-130871.

When the tumor marker is a nucleic acid, it is preferably to use a nucleic acid amplification method such as loop-mediated isothermal amplification (LAMP), and polymerase chain reaction (PCR). When the tumor marker is mRNA, before a nucleic acid amplification reaction, a nucleic acid amplification method comprising a reverse transcription reaction (e.g. RT-PCR method, RT-LAMP method etc.) can be used. When the tumor marker is mRNA, specifically, to the sample for measurement are added a primer, an RNA dependent DNA polymerase (reverse transcriptase), a DNA dependent DNA polymerase (hereinafter, simply also referred to as DNA polymerase) and the like to prepare a reaction solution, and nucleic acid amplification is performed. And, an amplified cDNA is detected.

The reverse transcription reaction and the nucleic acid amplification reaction may be appropriately changed in condition, depending on a sequence of cDNA and a sequence of a primer corresponding to a marker which is a template. Conditions of the reverse transcription reaction and the nucleic acid amplification reaction are described, for example, in Sambrook, J. et al. (1989) Molecular Cloning: A Laboratory Manual (2^{nd} ed.), Cold Spring Harbor Laboratory Press, New York.

A primer for detecting the tumor marker is not particularly limited in its sequence, as far as it is a polynucleotide which can amplify cDNA corresponding to the tumor marker. A length of the primer is preferably 5 to 100 nucleotides, more preferably 10 to 50 nucleotides. The primer can be prepared by the nucleic acid synthesis method which is known in the art.

As the reverse transcriptase and the DNA polymerase, those which are well-known in the art can be used. Examples of the reverse transcriptase include reverse transcriptase derived from AMV (Avian Myeloblastosis Virus), and reverse transcriptase derived from M-MLV (Molony Murine Leukemia Virus). And, as the DNA polymerase, a TaqDNA polymerase, a PfuDNA polymerase, a T4 DNA polymerase, and a Bst DNA polymerase can be used.

By measuring a nucleic acid amplification product produced by the nucleic acid amplification, information related to an absolute amount of a tumor marker is obtained. The tumor marker can be measured. When the tumor marker is mRNA, Quantitative Reverse Transcription-PCR (QRT-PCR), Quantitative Reverse Transcription-LAMP (QRT-LAMP) and the like are preferably used. According to these methods, the optical state (turbidity, absorbance, fluorescent intensity etc.) of the reaction solution is changed accompanying with amplification of cDNA which has been reverse-transcribed from mRNA. Therefore, the optical state of the reaction solution is measured at real time as information related to an absolute amount of a tumor marker, and is used in determination of malignant tumor metastasis.

As an example of QRT-PCR, the known method such as SYBR Green method (a method of adding SYBR Green to a reaction solution before a nucleic acid amplification reaction in advance, and measuring a fluorescent intensity which is increased accompanied with amplification of cDNA during an amplification reaction, at real time), and TaqMan (registered trade mark of Roche Diagnostic) can be used.

A measured value of the tumor marker can be calculated based on the number of cycles until a fluorescent intensity of the reaction solution reaches a predetermined value. As the number of the tumor marker in a sample is greater, a fluorescent intensity of the reaction solution reaches a predetermined value at a small number of cycles. As the number of the tumor marker in a sample is small, many cycle numbers are required until a fluorescent intensity of the reaction solution reaches a predetermined value.

Based on a time until a fluorescent intensity of the reaction solution reaches a predetermined value, a copy number of the tumor marker in a sample is calculated, and this can be compared with a threshold value corresponding to the copy number of the tumor marker. Based on this comparison result, metastasis of malignant tumor can be detected.

Alternatively, by comparing a time until a fluorescent intensity of the reaction solution reaches a predetermined value, with a threshold value corresponding to this without calculating the copy number, metastasis of malignant tumor may be detected.

When QRT-LAMP is used, a large amount of magnesium pyrophosphate is produced as a byproduct accompanied with amplification of cDNA. Since this magnesium pyrophosphate is insoluble, the reaction solution becomes cloudy accompanying with increase in magnesium pyrophosphate. Therefore, by optically measuring a turbidity (or absorbance) of the reaction solution at real time, the tumor marker can be measured. In addition, also in QRT-LAMP, the SYBR Green method can be used.

A measured value of the tumor marker can be calculated based on, for example, a time until a turbidity, an absorbance or a fluorescent intensity of the reaction solution reaches a predetermined value (detection time). As the number of the tumor marker in a sample is greater, the detection time is shorter. As the number of the tumor marker in a sample is smaller, the detection time is longer.

Based on the detection time, the copy number of the tumor marker in a sample can be calculated, and this may be compared with a threshold value. Based on this comparison result, metastasis of malignant tumor can be detected.

Alternatively, by comparing the detection time, with the threshold value corresponding to the detection time without calculating the copy number, metastasis of malignant tumor may be detected.

In the conventional molecular test of metastasis, metastasis of malignant tumor has been determined based on a value obtained by normalizing a measured value of the tumor marker.

In the molecular test, when a specimen substantially contains only an oncocyte, assuming that a housekeeping gene is expressed at a constant amount in any cell, malignant tumor metastasis can be correctly determined even when normalization is performed. However, when a normal cell is contained in a specimen, since expression level of housekeeping gene is increased in a specimen containing many normal cells when normalization is performed, possibility of metastasis is underestimated.

For example, when a specimen A containing many normal cells (an absolute amount of a tumor marker is 1, and expression level of a housekeeping gene is 1000), and a specimen B containing a smaller amount of normal cells than the specimen A (absolute amount of a tumor marker is 1, and expression level of a housekeeping gene is 10) are compared, if normalization is performed, the tumor marker of the specimen A is calculated as 1/1000, and the tumor marker of the specimen B is calculated as 1/10. When normalization is performed, although the tumor markers contained in the specimen A and the specimen B are equivalent, such determination result is obtained that a possibility of malignant tumor metastasis is lower in the specimen A in which the number of normal cells is larger. However, when studied without normalization as in the present embodiment, since tumor markers of the specimen A and the specimen B are equivalent, determination results regarding metastasis of malignant tumor of the specimen A and the specimen B become the same.

In addition, since only a part of a tissue can be tested by the conventional tissue diagnosis which is performed by microscopic test of a tissue section, there is a possibility that, when the section is prepared on a plane containing no oncocyte, the oncocyte is overlooked. On the other hand, in malignant tumor metastasis determination by a molecular test, since an entire specimen collected from a living body (or an extra specimen after preparation of a section) can be used, a possibility of overlooking is low unlike tissue diagnosis of testing only a part of a cross-section.

In the present embodiment, a measured value of mRNA of housekeeping gene of a specimen is not used for normalization, but may be used as a control for determining whether a nucleic acid amplification reaction has been precisely performed. Since the housekeeping gene is expressed in almost all kinds of cells, when mRNA of the housekeeping gene is detected, it is thought that a nucleic acid amplification reaction of the tumor marker has been also suitably performed. On the other hand, when mRNA of the housekeeping gene is not detected, it is thought that the nucleic acid amplification reaction has not been performed precisely, and a cause for inactivation of an enzyme is suspected.

Examples of the housekeeping gene include genes of **β**-actin, glyceraldehyde-3-phosphate dehydrogenase (GAPDH), **β**2 microglobin, hypoxanthine phosphoribosyltransferase 1 (HPRT1), and the like.

In the present embodiment, the aforementioned threshold value is a value which can be set depending on a kind of the tumor marker or the nucleic acid amplification method. The threshold value can be set, for example, at a value not more than a measured value of the tumor marker contained in a tissue or a cell for which malignant tumor metastasis has been confirmed (positive sample) and higher than a measurement value of the tumor marker contained in a tissue or a cell for which no malignant tumor metastasis has been confirmed (negative sample).

Alternatively, preferably, measured values of tumor markers of a plurality of positive samples, and measured values of tumor markers of plurality of negative samples are compared, and a value by which a positive sample and a negative sample can be discriminated at a highest provability can be adopted as a threshold value. For example, when metastasis of a breast cancer is detected using QRT-PCR, a threshold value can be set at 230 to 260 copies, when metastasis of a stomach cancer is detected, a threshold value can be set at 8 to 690 copies and, when metastasis of a colon cancer is detected, a threshold can be set at 79 to 180 copies. In addition, when metastasis of a breast cancer is detected using QRT-LAMP, a threshold value can be set at 86 to 360 copies, when metastasis of a stomach cancer is detected, a threshold value can be set at 10 to 270 copies and, when metastasis of colon cancer is detected, a threshold value can be set at 170 to 640 copies.

In the present embodiment, metastasis of the malignant tumor to a tissue or a cell is determined based on result of comparison between the absolute amount of a tumor marker and the threshold value. That is, when the absolute amount of a tumor marker is higher than the threshold value, it can be determined that metastasis is positive and, when the absolute amount is lower than the threshold value, it can be determined that metastasis is negative. By obtaining such the determination result, this can be an index for determination of technique, excision range, and therapeutic policy after operation.

In the present embodiment, a kind of the malignant tumor is not particularly limited. Examples include a breast cancer, a stomach cancer, an esophagus cancer, a colon cancer, a prostate cancer, leukemia and the like.

Detection of malignant tumor metastasis of the present embodiment can be performed by an apparatus.

This apparatus comprises a measuring assembly for obtaining information related to an absolute amount of a tumor marker, and a computer for obtaining the absolute amount of the tumor marker based on the information and detecting metastasis to the tissue by comparing the absolute amount with a predetermined threshold value.

A measuring assembly is not particularly limited as far as it can obtain information related to the absolute amount of a tumor marker, but a nucleic acid amplification apparatus which can measure a nucleic acid amplified by RT-LAMP method or RT-PCR method is preferable. The nucleic acid amplification apparatus is provided with a measurement part for measuring a nucleic acid amplification product obtained by amplifying the tumor marker in a tissue or a cell collected from a living body with using a primer and an enzyme.

A computer calculates the absolute amount of a tumor marker from data obtained at the measurement part, and compares this with a predetermined threshold value, thereby, detects metastasis of the malignant tumor. This apparatus may be provided with a display part for outputting detection result of malignant tumor metastasis.

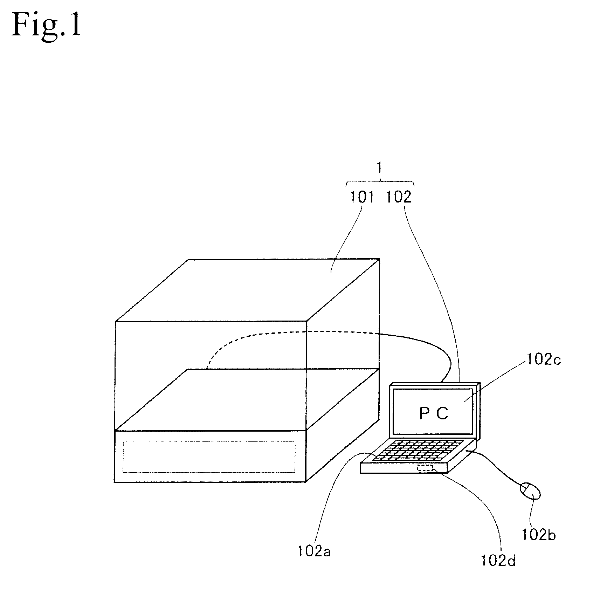

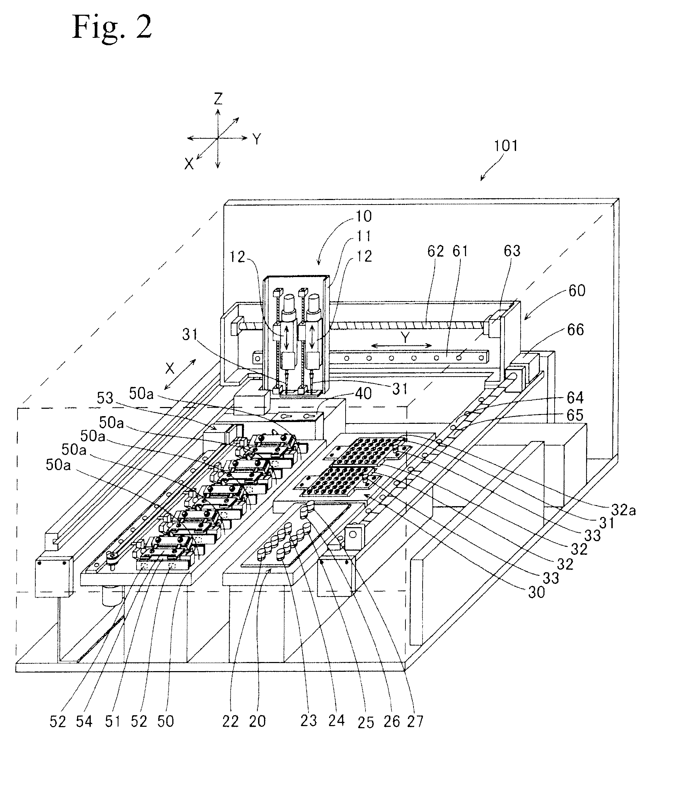

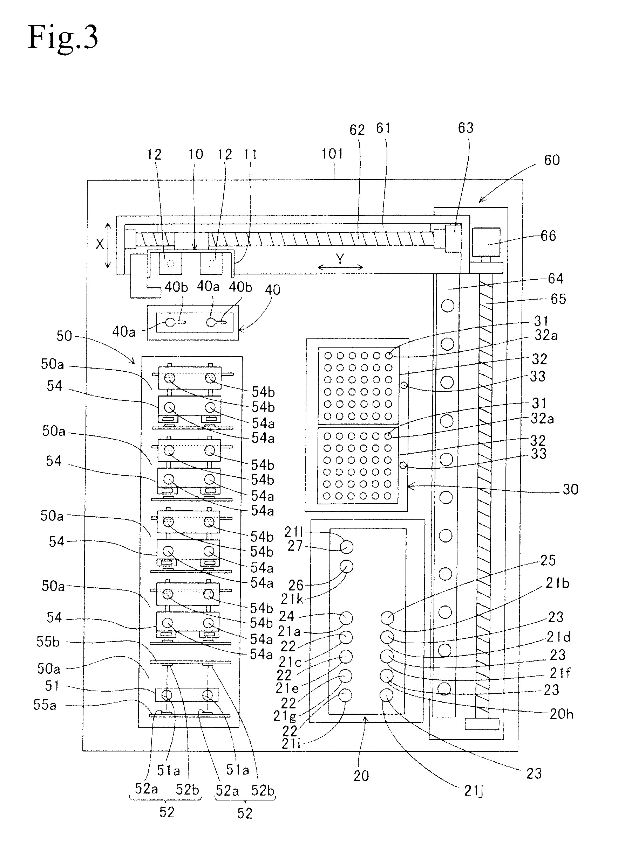

One embodiment of the apparatus of the present invention is shown in Figs 1 to 3. Fig.1 is a perspective showing an entire construction of the apparatus of the present embodiment. Fig.2 is a perspective showing an entire construction of a nucleic acid amplification device as a measurement means shown in Fig. 1 . Fig. 3 is a plane view of the nucleic acid amplification device of Fig. 2.

An apparatus of a certain embodiment of the present invention, as shown in Fig.1, can be constructed of a nucleic acid amplification device 101, and a personal computer (PC) 102 as a determination means, which is connected so that it can communicate with the nucleic acid amplification device by a wire or wireless system.

The nucleic acid amplification device 101, as shown in Fig.2, comprises a dispensing part 10, a sample setting part 20, a tip setting part 30, a tip discarding part 40, a reaction detection part 50 consisting of five reaction detection blocks 50a, and a transferring part 60 for transferring the dispensing part 10 in an X axis direction and a Y axis direction.

The dispensing part 10, as shown in Fig.2, comprises an arm part 11 which is moved in an X axis direction and a Y axis direction (horizontal direction) with the transference part 60, and duplicate (two) syringe parts 12 which can be moved independently relative to the arm part 11 in a Z axis direction (vertical direction).

As shown in Fig.2 and Fig. 3, in the sample setting part 20, ten sample container setting pores 21a to 21j, one enzyme reagent container setting pore 21k and one primer reagent container setting pore 211 are provided in an order from before the apparatus. And, ten sample container setting pores 21a to 21j are provided so as to be aligned in 5 rows and 2 columns. And, sample container setting pores 21c and 21d, sample container setting pores 21e and 21f, sample container setting pores 21g and 21h, and sample container setting pores 21i and 21j are provided at a sample setting position 1, a sample setting position 2, a sample setting position 3 and a sample setting position 4 in an order from an inner side of the apparatus, respectively.

In the present embodiment, a sample container 22 accommodating a solubilized extract solution (sample for measurement) prepared by treating (homogenizing, filtering etc.) an excised living body tissue (lymph node) in advance is set in sample container setting pores 21c, 21e, 21g and 21i on a front left side and, at the same time, a sample container 23 accommodating a diluted sample obtained by 10-fold diluting the above sample is set in sample container setting pores 21d, 21f, 21h and 21i on a front right side.

A container 24 accommodating a positive control for confirming that a nucleic acid to be amplified is normally amplified is mounted in the sample container setting pore 21a and, at the same time, a container 25 accommodating a negative control for confirming that a nucleic acid not to be amplified is not normally amplified is set in the sample container setting pore 21b.

An enzyme reagent container 26 accommodating a nucleic acid amplification enzyme reagent for amplifying cDNA corresponding to CK19mRNA (hereinafter, also referred to as CK19cDNA), and a primer reagent container 27 accommodating a primer reagent for CK19cDNA are set, respectively, in an enzyme reagent container setting pore 21k and a primer reagent container setting pore 211.

Each reaction detecting block 50a of a reaction detection part 50, as shown in Fig.2 and Fig.3, is constructed of a reaction part 51, two turbidity detection parts 52, and a lid closing mechanism part 53 (see Fig.2) . As shown in Fig.3, two detection cell setting pores 51a for setting a detection cell 54 are provided in a reaction part 51 provided on each reaction detection block 50a. Respective reaction detection blocks 50a are arranged at a cell setting position 1, a cell setting position 2, a cell setting position 3, a cell setting position 4 and a cell setting position 5 in an order from an inner side of the apparatus.

The turbidity detection part 52 is constructed of a LED light source part 52a consisting of blue LED having a wavelength of 465nm attached to a substrate 55a which is arranged on one side surface side of the reaction part 51, and a photodiode light receiving part 52b attached to a substrate 55b which is arranged on other side surface side of the reaction part 51. In each reaction detection block 50a, two sets of turbidity detection parts 52 are arranged, one set consisting of one LED light source part 52a and one photodiode light receiving part 52b.

The detection cell 54 has two cell parts 54a for accommodating a sample, and two lid parts 54b for closing two cell parts 54a.

A transferring part 60, as shown in Fig. 2, comprises a direct acting guide 61, a ball thread 62 for transferring the dispensing part 10 in a Y axis direction, a stepping motor 63 for driving the ball thread 62, a direct acting guide 64 and a ball thread 65 for transferring the dispensing part 10 in an X axis direction, and a stepping motor 66 for driving the ball thread 65. Transference of the dispensing part 10 in an X axis direction and a Y direction is performed by rotating ball threads 62 and 65 with stepping motors 63 and 66, respectively.

A personal computer 102, as shown in Fig.1, comprises a keyboard 102a and a mouse 102b for an inputting instrument, a display part 102c consisting of a monitor, and CPU 102d for analyzing the sample measurement result.

Then, referring to Fig.1 to Fig.3, operation of the determination apparatus 1 in accordance with the present embodiment will be explained. In this embodiment, as described above, cDNA is synthesized from mRNA of the tumor marker present in a lymph node tissue excised in malignant tumor operation, and this cDNA is amplified by the RT-LAMP method. Since a reaction solution is clouded with magnesium pyrophosphate generated accompanied with amplification, and a turbidity is increased, an amount of the tumor marker is measured by measuring a change in a turbidity of the reaction solution. This measurement result is compared with a threshold vale, thereby, metastasis of a malignant tumor to a lymph noted is determined.

First, as shown in Fig.2 and Fig.3, sample containers 22 accommodating a solution (hereinafter, referred to as sample) obtained by treating (homogenizing, filtering etc.) a tissue excised from a living body in advance to solubilize it are set in sample container setting pores 21c to 21j. Separately, a container 24 accommodating a positive control and a container 25 accommodating a negative control are set in sample container setting pores 21a and 21b (see Fig.3), respectively. Separately, an enzyme reagent container 26 accommodating a nucleic acid amplification enzyme reagent for amplifying CK19cDNA, and a primer reagent container 27 accommodating a primer reagent for amplifying CK19cDNA are set in an enzyme reagent container setting pore 21k (see Fig.3) and a primer reagent container setting pore 211, respectively. Separately, two racks 32 accommodating 36 disposable pipette tips 31 are arranged in tip setting parts 30, respectively.

When operation of the nucleic acid amplification apparatus 101 is started, first, an arm part 11 of a dispensing part 10 is moved to a tip setting part 30 from an initial position with a transferring part 60 shown in Fig.2, and two syringe parts 12 of the dispensing part 10 are moved downwardly in a tip setting part 30. Thereby, since tips of nozzle parts of two syringe parts 12 are pressed into upper opening parts of two pipette tips 31, pipette tips 31 are automatically mounted on tips of nozzle parts of two syringe parts 12. And, after two syringe parts 12 are moved upwardly, the arm part 11 of the dispensing part 10 is moved in an X axis direction towards above a primer reagent container 27 accommodating a primer reagent. And, after one syringe part 12 situated above the primer reagent container 27 is moved downwardly to suck the primer reagent, the one syringe part 12 is moved upwardly. Thereafter, the arm part 11 of the dispensing part 10 is moved with the transferring part 60 in a Y axis direction, so that other syringe part 12 is situated above the same primer reagent container 27. And, after other syringe part 12 is moved downwardly, and the primer reagent is sucked from the same primer reagent container 27, other syringe part 12 is moved upwardly. In such a way, the primer reagent in the primer reagent container 27 is sucked with two pipette tips 31 mounted on the syringe part 12.

After suction of the primer reagent, two syringe parts 12 are moved upwardly and, thereafter, the arm part 11 of the dispensing part 10 is moved with a transferring part 60 to above a reaction detection block 50a situated at a cell setting position 1 which is an innermost side (apparatus front inner side). And, in the reaction detection block 50a on an innermost side, by downward movement of two syringe parts 12, two pipette tips 31 mounted on two syringe parts 12 are inserted into two cell parts 54a of a detection cell 54, respectively. And, using the syringe part 12, the primer reagent is discharged into two cell parts 54a, respectively.

After discharge of the primer reagent, two syringe parts 12 are moved upwardly and, thereafter, the arm part 11 of the dispensing part 10 is moved with the transferring part 60 in an X axis direction towards above a tip discarding part 40. And, in the tip discarding part 40, the pipette tip 31 is discharged. Specifically, by downward movement of two syringe part 12, the pipette tip 31 is inserted into two tip discarding pores 40a (see Fig.3) of the tip discarding part 40. In this state, by movement of the arm part 11 of the dispensing part 10 with the transferring part 60 in a Y axis direction, the pipette tip 31 is moved to below a groove part 40b. And, since by upward movement of two syringe parts 12, a collar part on an upper side of the pipette tip 31 is abutted against a lower side on both sides of the groove part 40b, and undergoes a downward force from the lower side, the pipette tip 31 is automatically detached from a nozzle part of two syringe parts 12. Thereby, the pipette tip 31 is discarded into the tip discarding part 40.

Then, by the similar operation, an enzyme reagent is discharged into the cell part 54a from an enzyme reagent container 26 and, further, by the similar operation, a sample is discharged into the cell part 54a from a sample container 22 and a sample container 23.

And, after discharge of the primer reagent, the enzyme reagent and the sample into the cell part 54a is performed, a lid closing operation for a lid part 54b of a detection cell 54 is performed. After completion of this lid closing operation, when a liquid temperature in the detection cell 54 is elevated from about 20°C to about 65°C, cDNA is synthesized from a tumor marker (mRNA) by the RT-LAMP reaction and, further, the synthesized cDNA is amplified. Accompanying with amplification of cDNA, insoluble magnesium pyrophosphate is generated, and a reaction solution is clouded. Using a LED light source part 52a and a photodiode light receiving part 52b shown in Fig.3, turbidity in the detection cell 54 at an amplification reaction is detected (monitored).

Turbidity data of a sample is transmitted at real time from a nucleic acid amplification apparatus 101 to a personal computer 102. CPU 102d of the personal computer 102 receives turbidity data at real time, and measures a time until a turbidity reaches a predetermined value (detection time). Based on the measured detection time, a copy number of the tumor marker contained in the sample is calculated and, by comparing this with a predetermined threshold value, metastasis of a malignant tumor is determined.

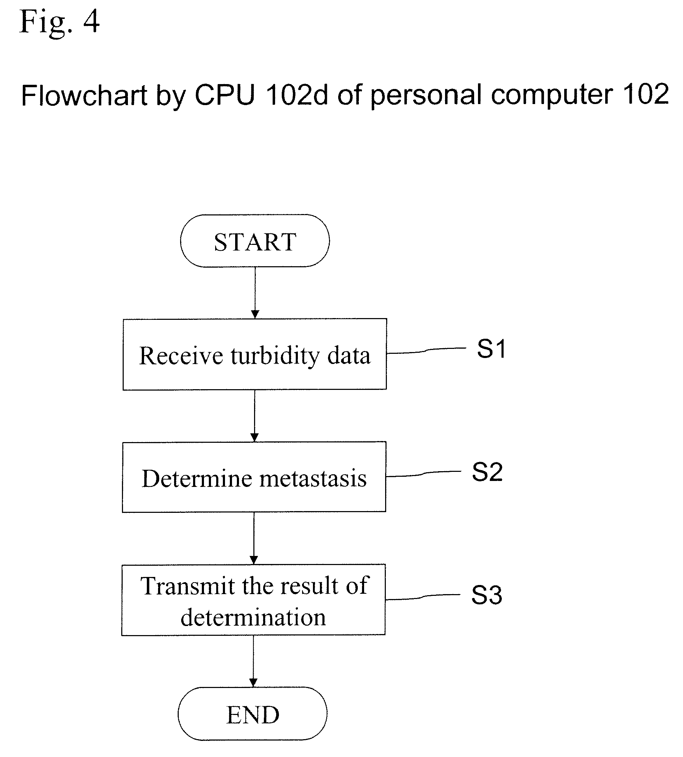

Herein, referring to Fig.4, processing by CPU 102d of the personal computer 102 will be explained.

In a step S1, CPU 102d receives turbidity data of the reaction solution from the nucleic acid amplification apparatus 101.

In a step S2, CPU 102d determines a detection time from received turbidity data, calculates a copy number of the tumor marker based on this detection time, and compares the copy number with a predetermined threshold value. Based on this comparison result, whether metastasis is positive or negative is determined. Specifically, when the copy number is equal to or more than a predetermined threshold value, malignant tumor metastasis is determined to be positive. When the copy number is less than a predetermined value, metastasis is determined to be negative.

In a step S3, CPU 102d transmits the result determined in the step S2 to a display part 102c.

In the aforementioned embodiment, the nucleic acid amplification apparatus 101 conducts measurement of turbidity data of a reaction solution and transmission of measured turbidity data to the personal computer 102, and the personal computer 102 conducts calculation of a copy number of a tumor marker, comparison between the copy number and the threshold value, and determination of metastasis. The nucleic acid amplification apparatus 101 may conduct measurement of turbidity data of the reaction solution, calculation of the copy number of the tumor marker, and transmission of the calculated copy number to the personal computer 102, and the personal computer 102 may conduct comparison between the copy number and the threshold value, and determination of metastasis.

### EXAMPLES

### <Example 1: Detection of breast cancer metastasis by QRT-PCR>

### Preparation of sample for measurement

Using 24 lymph nodes for which metastasis of an oncocyte derived from a breast cancer had been histologically recognized by tissue diagnosis (positive lymph node) and 52 lymph nodes for which metastasis of an oncocyte derived from a breast cancer had not been histologically recognized (negative lymph node), samples for measurement were prepared as follows.

To each lymph node (about 50 to 600 mg/node) was added 4 mL of a treatment solution (pH 3. 4; containing 200 mM glycine-HCl, 5% Brij35 (polyoxyethylene (35)lauryl ether (manufactured by Sigma-Aldrich Corporation)) and 20% DMSO (manufactured by Wako Pure Chemical Industries, Ltd.)), and this was homogenized with a blender.

The resulting homogenate was centrifuged at 10,000xg and room temperature for 1 minute.

Two hundred µL of the supernatant was collected.

RNA contained in the supernatant was purified using RNeasy Mini Kit (manufactured by Qiagen K.K., catalog No.74014), and the resulting solution was used as a sample for measurement.

### Measurement of tumor marker

Using samples for measurement prepared from a positive lymph node and a negative lymph node, QRT-PCR was performed with a real time PCR apparatus (ABI Prism (registered trade mark) 7000 Sequence Detection System, Applied Biosystems Japan Ltd.), and CK19mRNA was measured.

Real time RT-PCR was performed using Quanti Tect SYBR Green RT-PCR kit (manufactured by Qiagen K.K., catalog No.204245) which is a QRT-PCR kit according to an instruction book. A composition of a reaction solution and the PCR condition are as follows:
Primer for detecting CK19:
Forward primer: 5'-CAGATCGAAGGCCTGAAGGA-3' (SEQ ID No.: 1)
Reverse primer: 5'- CTTGGCCCCTCAGCGTACT-3' (SEQ ID No.: 2)

| Reaction solution: | |
|---|---|
| RNase free H₂O | 10.99 µL |
| 2xMaster mix | 12.50 µL |
| 100 µM forward primer (final concentration 500 nM) | 0.13 µL |
| 100 µM reverse primer (final concentration 500 nM) | 0.13 µL |
| Quanti Tect RT mix | 0.25 µL |
| Sample for measurement | 1.00 µL |
| Total | 25.00 µL |

PCR condition:
50°C, 30 minutes
95°C, 15 minutes
40 Cycles of the following steps;
94°C, 15 seconds
60°C, 1 minute

A PCR cycle number when a fluorescent intensity of the reaction solution exceeded Threshold (value automatically set by a SDS software loaded on the real time PCR apparatus) was obtained, and a mRNA copy number was calculated based on this PCR cycle number.

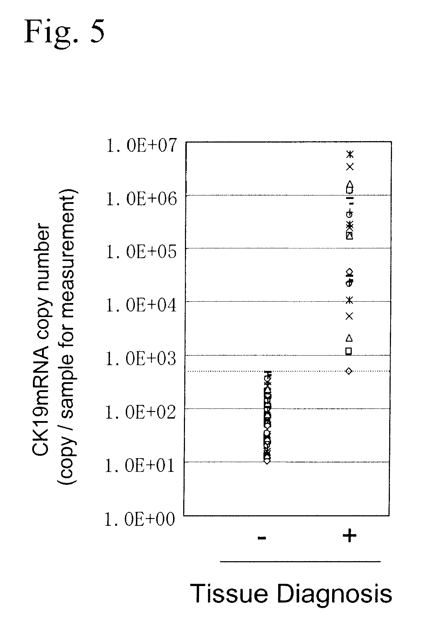

A threshold value was set at 500 copies per sample for measurement (dotted line in Fig.5).

The mRNA copy number of each sample for measurement is shown in Fig.5.

It is seen that, by the present example, a specimen (+) which was determined to be a positive sample in tissue diagnosis can be determined to be positive, and a specimen (-) which was determined to be a negative sample in tissue diagnosis can be determined to be negative.

### <Comparative Example 1>

As a housekeeping gene, an amount of mRNA of **β**-actin in a sample for measurement was measured. Measurement was performed according to the same manner as that of measurement of CK19mRNA in Example 1. Primers used in PCR were as follows.
Primer for detecting **β**-actin:
Forward primer: 5'- CCACACTGTGCCCATCTACG-3' (SEQ ID No.: 3)
Reverse primer: 5'- AGGATCTTCATGAGGTAGTCAGTCAG-3' (SEQ ID No.:4)

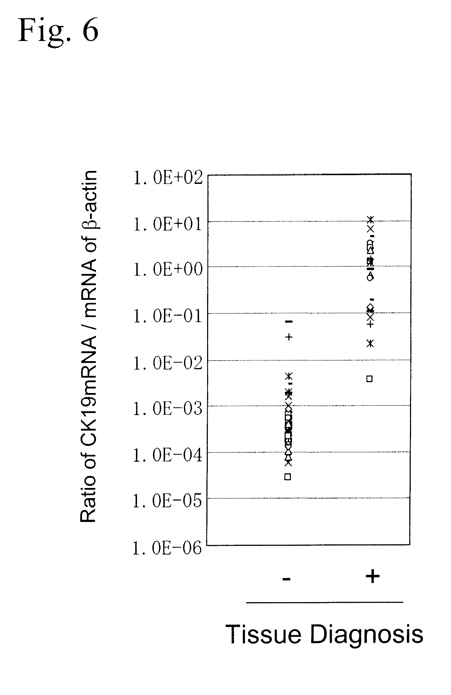

(Normalized) result obtained by dividing a copy number of CK19mRNA obtained in Example 1 by a copy number of mRNA of **β**-actin obtained in Comparative Example 1 is shown in Fig.6.

From the result of Fig.6, it is can be seen that, when a copy number of CK19mRNA is normalized, even if a threshold value is set between 0.0001 to 0.001, there is a possibility that a few negative samples are determined to be positive.

### <Example 2: Determination of metastasis of colon cancer by QRT-PCR>

According to the same manner as that of Example 1 except that 34 lymph nodes for which metastasis of an oncocyte derived from a colon cancer had been histologically recognized by tissue diagnosis (positive lymph node) and 40 lymph nodes for which metastasis of an oncocyte derived from a colon cancer had not been histologically recognized (negative lymph node) were used, an absolute amount of CK19mRNA in a sample for measurement was measured.

A PCR cycle number when a fluorescent intensity of a reaction solution exceeded Threshold was obtained, and a copy number of mRNA was calculated based on this PCR cycle number.

A threshold value was set at 130 copies per sample for measurement.

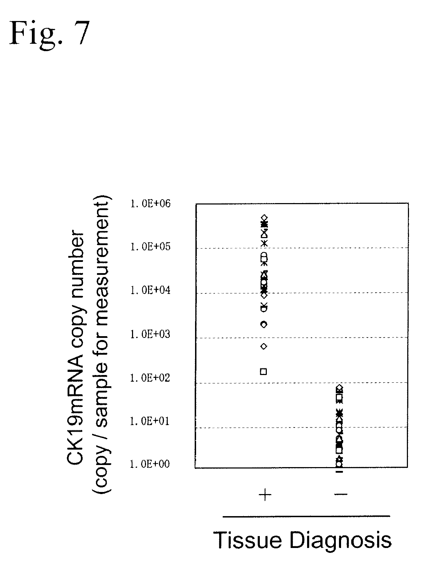

The mRNA copy number for each sample measurement is shown in Fig.7.

It can be seen that, according to the method of the present Example, a specimen (+) which was determined to be a positive sample by tissue diagnosis can be determined to be positive, and a specimen (-) which was determined to be a negative sample by tissue diagnosis can be determined to be negative.

### <Example 3: Determination of metastasis of stomach cancer by QRT-PCR>

According to the same manner as that of Example 1 except that 7 lymph nodes for which metastasis of an oncocyte derived from a stomach cancer had been histologically recognized by tissue diagnosis (positive lymph node) and 8 lymph nodes for which metastasis of an oncocyte derived from a stomach cancer had not been histologically recognized (negative lymph node) were used, an absolute amount of CK19mRNA was measured.

A PCR cycle number when a fluorescent intensity of a reaction solution exceeded Threshold was obtained, and a copy number of mRNA was calculated based on this PCR cycle number.

A threshold vale was set at 350 copies per sample for measurement.

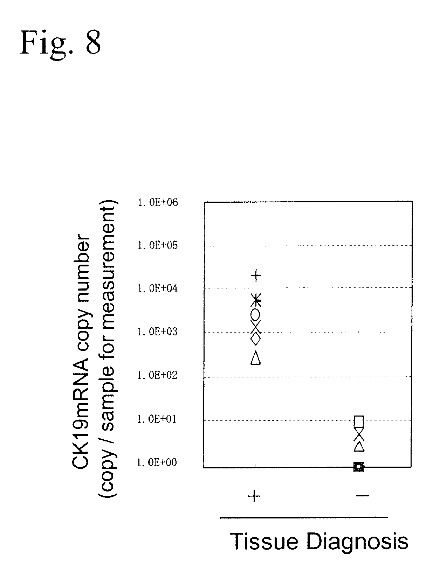

The copy number of mRNA of each sample for measurement is shown in Fig.8.

It can be seen that, according to methods of Examples 1 to 3, a specimen (+) which was determined to be a positive sample by tissue diagnosis can be determined to be positive, and a specimen (-) which was determined to be a negative sample by tissue diagnosis can be determined to be negative.

From the forgoing results, it was revealed that, according to methods of Examples 1 to 3, malignant tumor metastasis can be correctly determined regardless of a kind of a malignant tumor.

### <Example 4: Determination of metastasis of breast cancer by QRT-LAMP>

### Preparation of sample for measurement

Using 19 lymph nodes for which metastasis of an oncocyte derived from a breast cancer had been histologically recognized by tissue diagnosis (positive lymph node) and 45 of lymph nodes for which metastasis of an oncocyte derived from a breast cancer had not been histologically recognized (negative lymph node), a sample for measurement was prepared as follows.

To each lymph node (about 50 to 600 mg/node) was added 4 mL of a treatment solution (pH 3. 4; containing 200 mM glycine-HCl, 5% Brij35 (polyoxyethylene (35)lauryl ether, manufactured by Sigma-Aldrich Corporation) and 20% DMSO (manufactured by Wako Pure Chemical Industries, Ltd.)), and this was homogenized with a blender.

The resulting homogenate was centrifuged at 10,000xg and room temperature for 1 minute, and 200 µL of the supernatant was collected.

This supernatant was used as a sample for measurement.

### Preparation of reaction buffer

The following were mixed to prepare 13.97 **µ**l of a reaction solution.

| | |
|---|---|
| 750 nM Tris buffer (pH8.0) | 1.00 µl |
| 10×Thermopol buffer (manufactured by New England Biolaboratory) | 2.50 µl |
| 10 mM dNTPs | 2.00 µl |
| 100 mM MgSO₄ | 0.75 µl |
| 100 mM Dithiothreitol | 1.25 µl |
| 2% Tergitol (manufactured by Sigma Aldrich Japan K.K.) | 2.50 µl |
| H₂O | 3.97 µl |

### Preparation of enzyme reagent

The following respective ingredients were mixed to prepare 3.04 **µ**l of an enzyme reagent.

| | |
|---|---|
| 10U/µl AMV reverse transcriptase (manufactured by Promega K.K. Japan) | 0.14 µl |
| 8U/µl Bst DNA polymerase (manufactured by New England Biolaboratory) | 2.27 µl |
| RNase inhibitor (manufactured by Promega K.K. Japan) | 0.63 µl |

### Preparation of primer solution

The following respective ingredients were mixed to prepare 6.00 **µ**l of a primer reagent.

| | |
|---|---|
| 80 pmol/µl forward inner primer (SEQ ID No.5: 5'-GGAGTTCTCAATGGTGGCACCAACTACTACACGACCATCCA-3') | 1.00 µl |
| 80 pmol/µl reverse inner primer (SEQ ID No.6: 5'-GTCCTGCAGATCGACAACGCCTCCGTCTCAAACTTGGTTCG-3') | 1.00 µl |
| 5 pmol/µl forward outer primer (SEQ ID No.7: 5'-TGGTACCAGAAGCAGGGG-3') | 1.00 µl |
| 5 pmol/µl reverse outer primer (SEQ ID No.8: 5'-GTTGATGTCGGCCTCCACG-3') | 1.00 µl |
| 60 pmol/µl forward loop primer (SEQ ID No.9: 5'-AGAATCTTGTCCCGCAGG-3') | 1.00 µl |
| 60 pmol/µl reverse loop primer (SEQ ID No.10: 5'-CGTCTGGCTGCAGATGA-3') | 1.00 µl |

### Preparation of reaction solution

The above reaction buffer, enzyme reagent and primer solution were mixed to prepare 23 **µ**l of a solution. This was mixed with 2 **µ**l of a sample for measurement to prepare 25 **µ**l of a reaction solution.

### Nucleic acid amplification and measurement thereof by QRT-LAMP

Using a real time turbidimeter LA-200 (manufactured by Teramecs Co., Ltd.), amplification of a nucleic acid in a reaction solution, and clouding of the reaction solution due to magnesium pyrophosphate produced as a byproduct of nucleic acid amplification were measured at real time.

A time until a turbidity of the reaction solution reaches 0.1 (detection time) was measured.

Based on this detection time, a copy number (absolute amount) of CK19mRNA was calculated.

A threshold value was set at 220 copies.

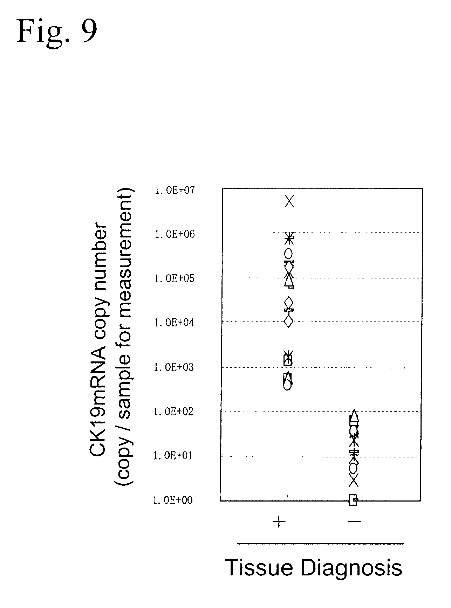

A copy number of mRNA of each sample for measurement is shown in Fig.9.

It can be seen that, according to the method of the present Example, a specimen (+) which was determined to be a positive sample by tissue diagnosis can be determined to be positive, and a specimen (-) which was determined to be a negative sample by tissue diagnosis can be determined to be negative.

### <Example 5: Determination of metastasis of colon cancer by QRT-LAMP>

According to the same manner as that of Example 4 except that 34 lymph nodes for which metastasis of an oncocyte derived from a colon cancer had been histologically recognized by tissue diagnosis (positive lymph node) and 40 lymph nodes for which metastasis of an oncocyte derived from a colon cancer had not been histologically recognized (negative lymph node) were used to prepare a sample form measurement, an absolute amount of CK19mRNA was measured.

A time until cDNA corresponding to CK19mRNA contained in each sample for measurement is amplified, and turbidity reaches 0.1 (detection time) was measured by QRT-LAMP.

Based on this detection time, a copy number (absolute amount) of CK19mRNA was calculated.

A threshold value was set at 400 copies.

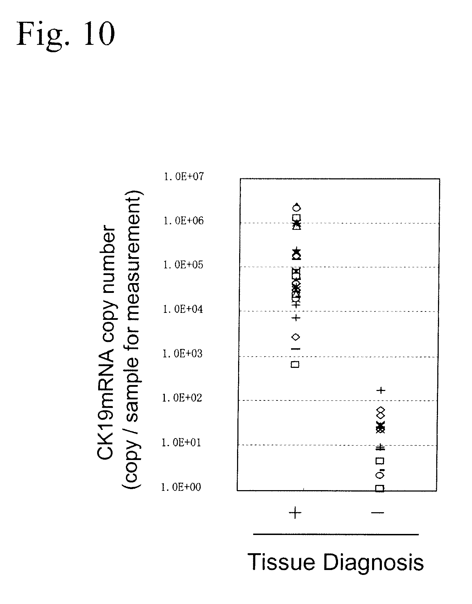

The copy number of mRNA of each sample for measurement is shown in Fig.10.

It can be seen that, according to the method of the present Example, a specimen (+) which was determined to be a positive sample by tissue diagnosis can be determined to be positive, and a specimen (-) which was determined to be a negative sample by tissue diagnosis can be determined to be negative.

### <Example 6: Determination of metastasis of stomach cancer by QRT-LAMP>

According to the same manner as that of Example 4 except that 7 lymph nodes for which metastasis of an oncocyte derived from a stomach cancer had been histologically recognized by tissue diagnosis (positive lymph node) and 8 lymph nodes for which metastasis of an oncocyte derived from a stomach cancer had not been histologically recognized (negative lymph node) were used to prepare a sample form measurement, an absolute amount of CK19mRNA was measured.

A time until cDNA corresponding to mRNA contained in each sample for measurement is amplified, and a turbidity reaches 0.1 (detection time) was measured by QRT-LAMP.

Based on this detection time, a copy number (absolute amount) of CK19mRNA was calculated.

A threshold value was set at 140 copies.

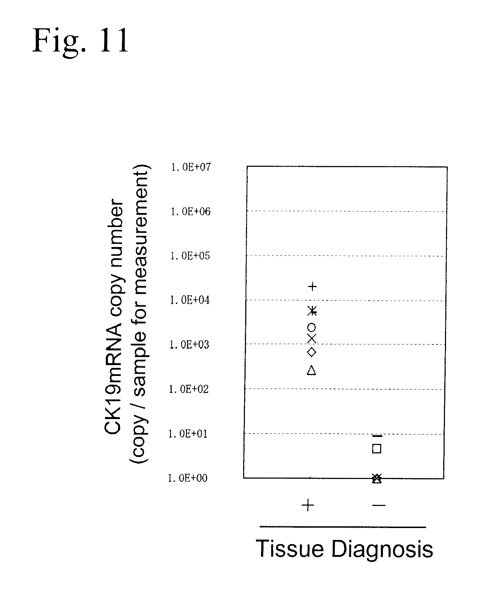

The copy number of mRNA of each sample for measurement is shown in Fig.11.

It can be seen that, according to methods of the Examples 4 to 6, a specimen (+) which was determined to be a positive sample by tissue diagnosis can be determined to be positive, and a specimen (-) which was determined to be a negative sample by tissue diagnosis can be determined to be negative.

From the forgoing results, it was revealed that, according methods of Examples 4 to 6, metastasis of malignant tumor can be correctly determined regardless of a kind of a malignant tumor.

From the forgoing, it was seen that, by using the absolute amount of a tumor marker without normalization, metastasis of malignant tumor is correctly determined. In addition, it was revealed that the determination method of the present invention can perform effective determination regardless of a kind of a malignant tumor and a kind of a nucleic acid amplification method.

## Claims

1. A method for judging the presence or absence of metastasis of malignant tumor, comprising steps of:
measuring an absolute amount of a tumor marker in the tissue or in a cell of the tissue obtained from a living subject; and
judging the presence or absence of metastasis of malignant tumor to the tissue by comparing the absolute amount with a predetermined threshold value.

2. The method according to claim 1, wherein the tissue is a lymph node.

3. The method according to claim 1 or 2, wherein the tumor marker is transferred from the tissue or the cell to a sample solution which is prepared by treating the tissue or the cell with a treatment solution, wherein the treatment solution comprises a dimethyl sulfoxide.

4. The method according to any of claims 1 to 3, wherein the tumor marker is an mRNA.

5. The method according to claim 4, wherein the measuring comprises:
synthesizing a cDNA from the mRNA;
amplifying the cDNA; and
obtaining the absolute amount of the tumor marker based on an amount of amplified cDNA.

6. The method according to claim 4 or 5, wherein the absolute amount of the tumor marker is a copy number of the mRNA.

7. The method according to any of claims 1 to 6, wherein the tumor marker is an mRNA of cytokeratin.

8. The method according to any of claims 1 to 7, wherein the absolute amount of the tumor marker is a number of molecule of the tumor marker.

9. The method according to any of claims 1 to 8, wherein the malignant tumor is selected from the group consisting of breast cancer, stomach cancer, esophageal cancer, colon cancer, and prostate cancer.

10. An apparatus for judging the presence or absence of metastasis of malignant tumor, comprising:
a measuring assembly for obtaining information related to an absolute amount of a tumor marker in the tissue or in a cell of the tissue obtained from a living subject; and
a computer for obtaining absolute amount of the tumor marker based on the information related to the absolute amount of the tumor marker, and judging the presence or absence of metastasis to the tissue by comparing the absolute amount of the tumor marker with a predetermined threshold value.

11. The apparatus according to claim 10, wherein
the measuring assembly comprises:
a synthesizing means for synthesizing a cDNA from the mRNA; and
an amplifying means for amplifying the cDNA,
wherein the information related to the absolute amount of the tumor marker is information related to an amount of amplified cDNA, and
the computer comprises:
a calculating means for calculating an absolute amount of the mRNA based on the information related to the amount of amplified cDNA; and
a comparing means for comparing the absolute amount of the mRNA with the predetermined threshold value.


SEQUENCE LISTING

<110>  Sysmex Corporation

<120>  A method and apparatus for judging the presence or absence of metastasis of malignant
tumor

<130>  SYSM-012-EP

<150>  JP 2006-163833
<151>  2006-06-13

<150>  JP 2006-355544
<151>  2006-12-28

<160>  10

<170>  PatentIn version 3.1

<210>  1
<211>  20
<212>  DNA
<213>  Artificial

<220>
<223>  Primer

<400>  1
cagatcgaag gcctgaagga                                                    20


<210>  2
<211>  19
<212>  DNA
<213>  Artificial

<220>
<223>  Primer

<400>  2
cttggcccct cagcgtact                                                     19


<210>  3
<211>  20
<212>  DNA
<213>  Artificial

<220>
<223>  primer

<400>  3
ccacactgtg cccatctacg                                                    20


<210>  4
<211>  26
<212>  DNA
<213>  Artificial

<220>
<223>  Primer

<400>  4
aggatcttca tgaggtagtc agtcag                                             26


<210>  5
<211>  41
<212>  DNA
<213>  Artificial

<220>
<223>  Primer

<400>  5
ggagttctca atggtggcac caactactac acgaccatcc a                            41

<210>  6
<211>  41
<212>  DNA
<213>  Artificial

<220>
<223>  Primer

<400>  6
gtcctgcaga tcgacaacgc ctccgtctca aacttggttc g                41


<210>  7
<211>  18
<212>  DNA
<213>  Artificial

<220>
<223>  Primer

<400>  7
tggtaccaga agcagggg                                          18


<210>  8
<211>  19
<212>  DNA
<213>  Artificial

<220>
<223>  Primer

<400>  8
gttgatgtcg gcctccacg                                         19


<210>  9
<211>  18
<212>  DNA
<213>  Artificial

<220>
<223>  Primer

<400>  9
agaatcttgt cccgcagg                                          18


<210>  10
<211>  17
<212>  DNA
<213>  Artificial

<220>
<223>  Primer

<400>  10
cgtctggctg cagatga                                           17